# EUROPEAN PATENT APPLICATION

(11) **EP 1 358 893 A1**
(43) Date of publication of application: **05.11.2003**
(21) Application number: 02252895.4
(22) Date of filing: 24.04.2002
(51) Int. Cl.: A61L 15/38

(54) **Skin dressings containing an oxidoreductase and a peroxidase**

(71) Applicant: Insense Limited, Sharnbrook, Bedford MK44 1LQ (GB)
(72) Inventor: The designation of the inventor has not yet been filed
(74) Representative: Matthews, Heather Clare

(57) **Abstract**

A skin dressing, particularly a wound dressing, comprises oxidoreductase enzyme and peroxidase enzyme, wherein the enzymes are present in hydrated condition, e.g. being present in one or more hydrated gels. The dressing is used by being located on the skin of a human or animal e.g. over a wound. The oxidoreductase enzyme catalyses a reaction that produces hydrogen peroxide from an appropriate substrate, the substrate either being naturally present in body fluids and/or being supplied separately and/or being incorporated into the dressing. The currently preferred oxidoreductase enzyme is glucose oxidase. This catalyses reaction of β-D-glucose substrate to give hydrogen peroxide and gluconic acid. A mixture of oxidoreductase enzymes may be used. The hydrogen peroxide resulting from the reaction catalysed by the oxidoreductase enzyme undergoes reaction catalysed by the peroxidase enzyme to produce a variety of species including reactive oxygen intermediates that have antimicrobial properties and that can therefore assist in promoting wound healing.

## Description

### Field of the Invention

This invention relates to skin dressings for application to a part of a human or animal body for treatment of skin, particularly (but not exclusively) wound dressings for treatment of compromised skin, particularly skin lesions, i.e. any interruption in the surface of the skin, whether caused by injury or disease, including ulcers, burns, cuts, punctures, lacerations, blunt traumas etc.

### Background to the Invention

Wounds frequently become infected. Wound dressings may carry antiseptic substances, and the physical protection they provide prevents ingress of extra infecting microbes, although this microbial exclusion is seldom absolute. Antiseptic substances carried on the dressing pad are not usually very effective, possibly because they do not readily diffuse into the wound at a steady rate. Moreover, the most effective substances, antibiotics, are not available for routine use, because of the ever-present problems of emerging drug resistance.

US4576817 proposes a bacteriostatic fibrous wound dressing incorporating dry enzymes such as glucose oxidase and lactoperoxidase to generate e.g. hydrogen peroxide and hypoiodite on contact with serum.

WO 01/28600 discloses a wound dressing including dry glucose oxidase, dry lactoperoxidase and an iodide salt in a polymeric gel matrix. The glucose oxidase catalyses an oxidation reaction of glucose present in body fluids of a wound site to generate hydrogen peroxide. The action of lactoperoxidase on hydrogen peroxide and iodide generates elemental iodine, which is a powerful anti-infective agent.

### Summary of the Invention

The present invention provides a skin dressing comprising oxidoreductase enzyme and peroxidase enzyme, wherein the enzymes are present in hydrated condition.

By providing the enzymes in hydrated condition, the enzymes are present in a wet, active state in the dressing and can begin functioning immediately when brought into contact with appropriate substrate on use of the dressing. This is to be contrasted with prior art dressings where enzymes are in dry condition and require initial hydration on use, thus delaying enzyme functioning and consequent antimicrobial effects.

The dressing is used by being located on the skin of a human or animal e.g. over a wound or on a region of skin to be treated for cosmetic or therapeutic purposes, e.g. for treatment of acne or other skin conditions. The oxidoreductase enzyme catalyses a reaction that produces hydrogen peroxide from an appropriate substrate, the substrate either being naturally present in body fluids and/or being supplied separately and/or being incorporated into the dressing. Oxidoreductase enzymes suitable for use in the invention and the corresponding substrates (which are present in blood and tissue fluids) include the following:

| Enzyme | Substrate |
|---|---|
| Glucose oxidase | β-D glucose |
| Hexose oxidase | Hexose |
| Cholesterol oxidase | Cholesterol |
| Galactose oxidase | D-galactose |
| Pyranose oxidase | Pyranose |
| Choline oxidase | Choline |
| Pyruvate oxidase | Pyruvate |
| Glycollate oxidase | Glycollate |
| Aminoacid oxidase | Aminoacid |

The currently preferred oxidoreductase enzyme is glucose oxidase. This catalyses reaction of β-D-glucose substrate to give hydrogen peroxide and gluconic acid.

A mixture of oxidoreductase enzymes may be used.

The hydrogen peroxide resulting from the reaction catalysed by the oxidoreductase enzyme undergoes reaction catalysed by the peroxidase enzyme to produce a variety of species including reactive oxygen intermediates that have antimicrobial properties and that can therefore assist in promoting wound healing.

Peroxidase enzymes useful in the invention include lactoperoxidase, horseradish peroxidase, iodide peroxidase, chloride peroxidase and myeloperoxidase, with lactoperoxidase currently being favoured.

A mixture of peroxidase enzymes may be used.

The active species produced by the action of peroxidase are difficult to define, and will to some extend depend the particular peroxidase in question. For example, horse radish peroxidase works very differently to lactoperoxidase. The detailed chemistry is complicated by the fact that the products are so reactive that they rapidly give rise to other, associated products that are also very reactive. It is believed that hydroxyl radicals, singlet oxygen and superoxide are produced, just as in the "oxidative burst" reactions identified in neutrophil and macrophage leukocytes of the human body.

The enzymes are conveniently present in one or more hydrated gels.

The hydrated gel conveniently comprises an alginate gel, e.g. formed from alginic acid cross-linked in known manner, e.g. by use of calcium chloride. Such gels form an entrapping biopolymer matrix that retains the enzyme within the gel, preventing release into a wound in use of the dressing. The gel may be in the form of beadlets, beads, slabs or extruded threads etc.

The hydrated gel is desirably cast around a mechanical reinforcing structure, such as a sheet of cotton gauze or an inert flexible mesh, e.g. to providing a structurally reinforced hydrogel layer or slab.

The hydrated gel may alternatively be in the form of a shear-thinning gel, e.g. of suitable gums such as xanthan gum (e.g. available under the Trade Mark Keltrol), in this case preferably without a mechanical reinforcing structure. Such gums are liquid when subjected to shear stress (e.g. when being poured or squeezed through a nozzle) but set when static. Thus the gel may be in the form of a pourable component, facilitating production of gels in the dressing. Such a shear-thinning gel may also be used in combination with a preformed, mechanically reinforced gel.

The enzymes may be present in a gel in a number of possible forms, including in solution as free molecules. To improve efficiency of retention of the enzymes in the gel, the enzymes may be chemically conjugated to each other, chemically conjugated to other molecules (e.g. polyethylene imine), or incorporated in a solid support such as beads.

Gels of different types, e.g. cross-linked alginate and shear-thinning, may be used together in a single dressing.

The enzymes are preferably immobilised so they can be prevented from being released into a wound, where they would have the potential to trigger undesirable allergic responses (being generally derived from non-human sources, e.g. with most commercially available glucose oxidase being derived from the fungus *Aspergillius niger* and with lactoperoxidase typically being extracted from bovine milk) and would also be susceptible to degradation by the effect of proteases present in a wound.

The enzymes may be immobilised in known manner, e.g. by being irreversibly bound to a solid support such as a particle, bead or fibre, e.g. of cellulose, silica, polymer etc., using coupling methods known to those skilled in the art. Incorporating the enzymes in a cross-linked alginate gel as discussed above, e.g. in the form of beadlets, slabs or extruded threads, also has the effect of immobilising the enzymes. Known encapsulation techniques using polyamide are also appropriate.

The two enzymes are preferably located in separated hydrated gels, with the oxidoreductase enzyme located in a first hydrated gel and the peroxidase located in a second hydrated gel.

The first and second hydrated gels can be located in different regions of the dressing as required.

It is preferred that first gel (with oxidoreductase enzyme) is located in the vicinity of the outer parts of the dressing, i.e. remote from the skin in use, where oxygen levels are highest, with the second gel (with peroxidase enzyme) being located in the vicinity of the inner parts of the dressing, i.e. adjacent the skin in use, so that the antimicrobial species (at least some of which are very short lived due to their extreme reactivity) produced thereby are close to the skin and are not expended before they reach the desired site of action. The dressing thus preferably has a layered, stratified construction, comprising an upper (outer) layer of the first gel and a lower (inner) layer of the second gel.

The dressing desirably includes a layer of barrier material at the interface with the skin in use, i.e. adjacent the second gel (with peroxidase enzyme) in the preferred arrangement above, to prevent undesirable ingress to the dressing of catalase from the skin, e.g. from wound fluid or from microbes inhabiting the area. Ingress of catalase to the dressing is undesirable as this enzyme would compete with the peroxidase for reaction with hydrogen peroxide, thus reducing efficiency. Suitable barrier material includes, e.g. a semi-permeable sheet or membrane with a high molecular weight cut-off that is permeable to the antimicrobial species produced by the peroxidative peroxidase but impermeable to catalase. Suitable materials are known to those skilled in the art and include cellulose acetate film such as that used to make dialysis membranes with a molecular weight cut-off of about 15 kD.

The dressing desirably includes a source of substrate for the oxidoreductase enzyme, e.g. glucose for glucose oxidase. Glucose is conveniently in the form of honey which naturally provides other benefits such as healing and antimicrobial factors. The substrate must be physically separated from the oxidoreductase enzyme prior to use of the dressing, to prevent premature reaction. This will be discussed below. The substrate may be contained within an enclosure of a semi-permeable membrane material, e.g. material such as those used as dialysis membranes e.g. cellulose acetate (which is also often known as "visking tubing"), or within a gel slab or pad, e.g. of agarose. Such a gel slab or pad is desirably cast around a mechanical reinforcing structure, such as a sheet of cotton gauze etc., as discussed above. The substrate may conveniently be in the form of a shear-thinning gel, e.g. of a suitable gum such as xanthan gum as discussed above, preferably without a mechanical reinforcing structure, for pourable production.

In the preferred dressing of layered construction, as mentioned above, the source of substrate is preferably located (in use of the dressing) sandwiched between the upper layer of first gel containing oxidoreductase enzyme and the lower layer of second gel containing peroxidase enzyme. In this case, oxidoreductase enzyme may optionally also be included in the second gel to oxidise substrate that is liable to diffuse towards the second gel. Such oxidation is dependent on the presence of oxygen, which is in restricted supply at this location, with the oxidation reaction being proportional to the available oxygen.

In another alternative arrangement the peroxidase enzyme and substrate are both present in the second hydrated gel. In this case, the first hydrated gel (with oxidoreductase enzyme) is desirably located above and/or below the second gel in a layered arrangement.

Yet another option is to have a 4 layered structure, with an upper layer (remote from the skin) of first hydrated gel overlying a layer of substrate that overlies a further layer of first hydrated gel, in turn overlying a second hydrated gel layer at the bottom (adjacent the skin in use).

By providing an excess of substrate, so the dressing is able to function in use to generate antimicrobial species over an extended period of time, typically at least 2 days, particularly where enzyme-containing hydrated gel or gels are tightly cross-linked so retarding flow of substrate to the enzymes.

The dressing desirably also includes a supply of iodide, e.g. potassium iodide or sodium iodide, that will undergo peroxidase enzyme-catalysed reaction with hydrogen peroxide in known manner to produce hypoiodous acid and elemental iodine, which are known powerful antimicrobial agents, e.g. as discussed in WO 01/28600. The supply of iodide is conveniently located with the source of substrate for the oxidoreductase enzyme, as discussed above. However, even in the absence of iodide, antimicrobial active intermediates are still formed, as discussed above.

The dressing conveniently includes a covering or outer layer for adhering the dressing to the skin of a human or animal subject (in known manner). At least part of the covering should be of oxygen-permeable material to enable oxygen from ambient air to pass through the covering and enter into the body of the dressing in use, where it is required as a cosubstrate of the oxidoreductase catalysed reaction. The oxygen-permeable material may be in the form of a "window" set into an otherwise relatively oxygen-impermeable covering, e.g. of possibly more robust material.

Optionally the covering includes a window (or further window) in or through which can be seen indicator means e.g. an indicator sheet or similar structure that indicates (e.g. by changing colour) when the dressing chemistry is active. A further indicator may optionally be provided, which indicates (e.g. by changing colour) when the dressing chemistry has expired.

A further useful option is to provide immobilised catalase enzyme on the inner surface of the covering (e.g. secured to adhesive thereof). This will function rapidly to break down any excess hydrogen peroxide which may escape from a wound area. This feature will prevent potentially damaging build-up of hydrogen peroxide in areas of normal, undamaged skin.

The dressing may be supplied as a multi-part system, with different elements separately packaged, for assembly and use by an end user in accordance with supplied instructions. In particular, in embodiments including a source of substrate, e.g. glucose, this may be supplied packaged separately from other components, particularly the oxidoreductase enzyme, to prevent premature oxidation reaction. Alternatively, the dressing may be foldable. A typical embodiment of this sort comprises a linear arrangement of linked slabs or panels including a slab of the first hydrated gel, a slab of the second hydrated gel and a slab of substrate, with adjacent slabs linked by a respective hinge portion. Hydrophobic barrier material, e.g. a wax, is desirably impregnated into the hinge portions to prevent lateral diffusion Good results have been obtained with an embodiment comprising, in sequence, a slab of substrate, a slab of second hydrated gel (containing peroxidase enzyme) and a slab of first hydrated gel (containing oxidoreductase enzyme), with adjacent slabs linked by a respective foldable hinge portion. To prepare such a dressing for use the two outer slabs are folded in, to bring the slab of substrate so as to overly the slab of second gel and to bring the slab of first gel so as to overly the slab of substrate, thus forming a layered arrangement as described above. The resulting layered arrangement is placed on skin e.g. on a wound, with the slab of second gel in contact with the skin, and may be held in place e.g. by use of an adhesive covering.

Dressings of layered construction comprising shear-thinning gels can be readily produced, e.g. by an end user, by pouring or dropping the gels one on top of the other in appropriate order to produce a desired layered assembly of gels. Thus the different dressing component gels may be supplied in separate containers e.g. tubes or bottles or possibly a multicompartment jar. The different gels may be colour-coded with appropriately coloured latex for ease of identification. The gels may be applied directly to the skin of a user. A covering or outer layer may not be required with such embodiments.

Dressings in accordance with the invention (or components thereof) are suitably supplied in sterile, sealed, water-impervious packages, e.g. foil pouches.

Dressings in accordance with the invention can be manufactured in a range of different sizes and shapes for treatment of areas of skin e.g. wounds of different sizes and shapes. Appropriate amounts of enzymes, and substrates and iodide if present, for a particular dressing can be readily determined by experiment.

The invention will be further described, by way of illustration in the following Examples and with reference to the accompanying drawings, in which:

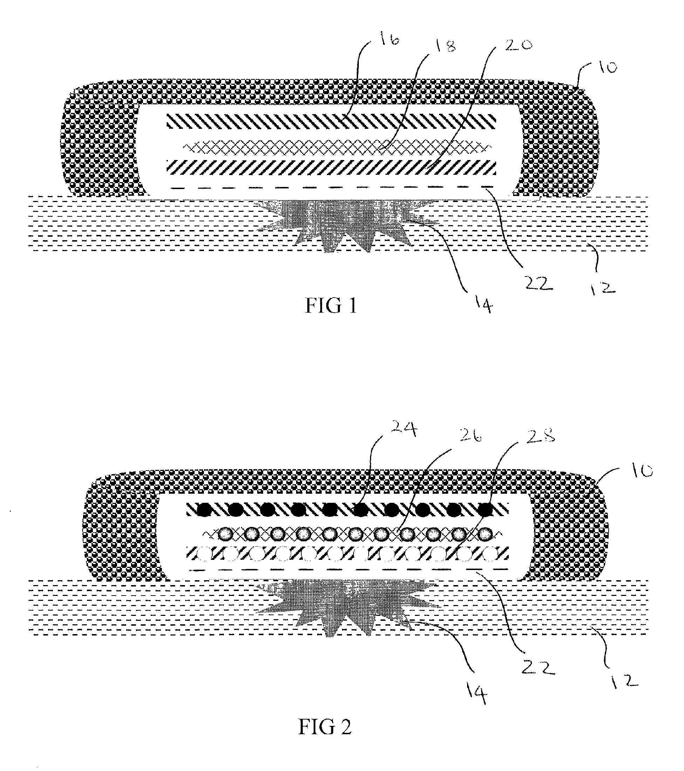

Figures 1 to 4 are schematic sectional illustrations of 4 different embodiments of wound dressings in accordance with the invention.

### Examples

### Construction of glucose oxidase and lactoperoxidase beadlets.

The enzyme, either lactoperoxidase (LPO) (from Sigma, cat no L2005) or glucose oxidase (GOX) (from Boehringer Mannheim, Cat No. 105147) is dissolved in pure water at the rate of 1 microgram per ml (LPO) or 10 micrograms per ml (GOX). A solution of alginic acid (Manucol DM (Manucol DM is a Trade Mark) from C P Kelco) (1 gram per 100 ml of water) is prepared at elevated temperature and cooled. Enzyme solution is mixed with the cooled alginic acid at the appropriate rate. The resulting alginic acid/enzyme solution is then pumped through a peristaltic pump into a tube which leads to an exit nozzle conveniently formed by a standard laboratory glass pasteur pipette, placed over a setting bath of calcium chloride solution (10% w/v). The flow of the pump and height of the exit nozzle are adjusted so that the outflowing stream of alginic acid/enzyme solution forms into discrete droplets as it enters the calcium chloride solution. Each droplet rapidly starts to solidify as the calcium begins to cross-link the alginic acid molecules and, 10 minutes after the delivery of the last droplet, the setting process is complete. All the newly formed beadlets are removed from the calcium chloride solution by pouring the whole through a sieve of suitable mesh size. Residual calcium chloride is removed by rinsing with pure water. The beadlets are stored in water or in water-tight containers, or in a physiological buffer solution such as phosphate buffered saline. Alternatively, they can be placed in glycerol or glycerol in water solutions to decrease the entrained water content. Whatever storage conditions are used, they must not be allowed to dry out or harden in the absence of water or glycerol.

### Construction of glucose beadlets.

The method described above is followed, except that no enzymes are added and glucose is included at the rate of 12.5g per 100ml in both the pure water and the calcium chloride setting bath. The gel beadlets are washed with calcium-free glucose solution, allowing removal of excess calcium without depleting the glucose.

### Preparation of an enzyme beadlet-containing pad.

Appropriately sized cotton lint is placed on a suitable surface through which water can flow (e.g. a flat plastic mesh). A suspension of enzyme-beadlets is poured onto the cotton lint, in such a way that the beadlets become entrapped in the raised nap of the fabric, as the suspending water flows away. A second piece of lint is then placed over the first, so as to sandwich the enzyme beadlets between the two fabric layers. Adhesive or stitching or stapling may be used to secure the top layer to the bottom layer. Excess fluid is drained away, but the pad is not allowed to dry out.

The number of beadlets contained per pad should be determined on the basis that each oxidase pad should carry about 100mg of GOX, and each peroxidase pad should carry about 10mg of LPO.

### Preparation of a pad containing glucose beadlets and iodide

The method described above is used with glucose-containing beadlets to prepare a pad containing entrapped glucose, except that an extra step is included at the end of the process, in which the pad is soaked in a solution of potassium iodide (10mM).

### Preparation of a glucose/iodide containing sachet.

Glucose is dissolved in a 5mM aqueous solution of potassium iodide at the rate of 12.5g per 100ml. This solution is then placed in a dialysis bag (previously placed in boiling water for 10 mins and thoroughly rinsed) with an accessible area of about 40x20 mm, and sealed.

### Demonstration of the oxidative activity of an assembled composite dressing.

A 1% aqueous solution of agarose is prepared, with potassium iodide added at a concentration of 10mM and soluble starch at concentration of 1% w/v. The solution is melted and dispensed into a petri dish to form a continuous layer about 5mm thick, and allowed to set. Once set a peroxidase pad as described above is laid on the surface, followed by a glucose pad or sachet as described above laid on top of that, and finally a glucose oxidase pad as described above is laid on top of them both to form a three-layered stack. The development of a blue colour within the starch agar indicates the oxidative activity of the composite dressing.

### Use of the assembled composite dressing as a wound treatment.

The pads or sachets for this purpose prepared as described above are sealed in appropriate pouches and subjected to gamma irradiation to ensure microbiological sterility, using techniques known and routinely used in the industry.

Firstly the wound is covered by a thin sheet of sterile gauze. Next the three layers of sterile pads are added as a thin stack, with the peroxidase pad first followed by the glucose pad or sachet next and the glucose oxidase pad last. The pads are cut to a size that just covers the open wound. Finally, the composite dressing is preferably held in place by adhesive film, such as normal "sticking plaster" or "Micropore" surgical tape.

### Other embodiments.

The enzymes can be immobilised on various types of particle or fibre, using coupling methods known by those skilled in the art. The particles can be made of cellulose, silica or various harmless polymers. Alginate can be used in forms other than beadlets, such as slabs or extruded threads, still using calcium as a setting agent. Microcapsules, such as those made by known techniques with polyamide, can be used to encapsulate each of the components.

Referring to the drawings, Figures 1 to 4 illustrate schematically various different embodiments of wound dressings in accordance with the invention. In all of the drawings, a cross-hatched element represents a sachet of semi-permeable membrane e.g. cellulose acetate or gel slab containing an aqueous solution of glucose and potassium iodide; an element with hatching lines extending from upper left to lower right represents a hydrated gel slab containing glucose oxidase trapped in the gel; and an element with hatching lines extending from upper right to lower left represents a hydrated gel slab containing lactoperoxidase trapped in the gel.

Filled circles represent beadlets of alginate gel (typically about 2mm in diameter) containing entrapped glucose oxidase. Alternatively other gels (e.g. agarose) or polymers could be used to form the beadlets. Glucose is able to diffuse into these beadlets and hydrogen peroxide is able to diffuse out.

Empty circles represent beadlets of alginate gel (typically about 2mm diameter) containing entrapped lactoperoxidase. Alternatively other gels (e.g. agarose) or polymers could be used to form the beadlets. Hydrogen peroxide is able to diffuse into these beadlets and reactive oxygen species are able to diffuse out.

Shaded circles represent beadlets of alginate gel (typically about 2mm diameter) containing entrapped glucose and potassium iodide. Alternatively, other gels (e.g. agarose) or polymers could be used to form the beadlets. Glucose is able to diffuse out of these beadlets.

Figure 1 illustrates one preferred embodiment of wound dressing in accordance with the invention. The dressing is of layered construction and comprises an outer layer or covering 10 in the form of an oxygen-permeable self-adhesive plaster, suitable for adhering to the skin 12 of a subject, so as to cover a wound 14. Covering 10 encloses an upper layer comprising a first moist pad 16 with immobilised glucose oxidase, an intermediate layer comprising a solution of glucose and potassium iodide in a semi-permeable sachet or gel slab 18 and a lower layer comprising a second moist pad 20 with immobilised lactoperoxidase. Below pad 20 is a sheet 22 of gauze, for contact with the wound 14. The pads and sachet may be generally as described in the Examples above.

The dressing is initially supplied as a multi-part system, with the individual components separately packaged in respective sealed, sterile packages. When required for use, the dressing components are removed from the packages and applied to a wound in appropriate manner and order to produce the final dressing as shown.

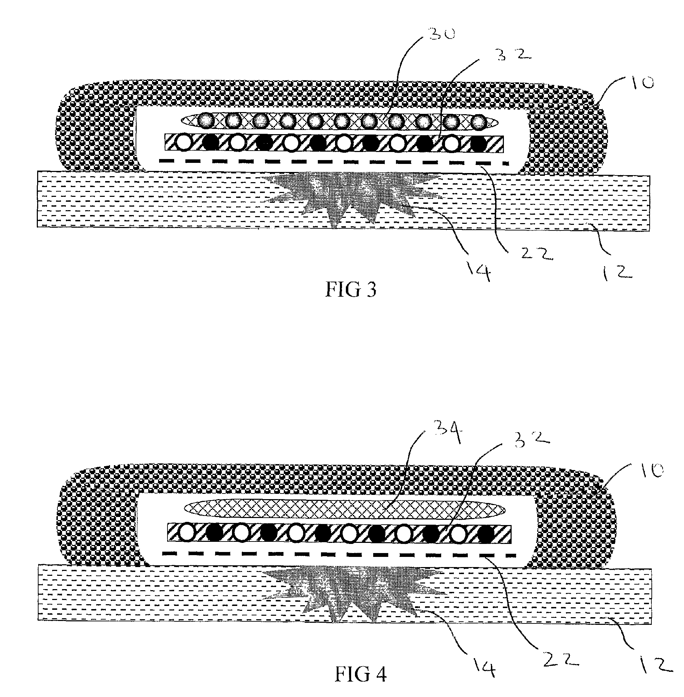

Figure 2 illustrates another preferred embodiment of wound dressing, generally similar to Figure 1, with similar components being identified by similar reference numerals. In this embodiment the upper layer comprises a first moist pad 24 with calcium alginate beads containing entrapped glucose oxidase. The intermediate layer comprises a pad 26 with gel beads containing glucose and potassium iodide. The lower layer comprises a second moist pad 28 with calcium alginate beads containing entrapped lactoperoxidase.

Figure 3 illustrates a further generally similar embodiment, but comprising an upper layer in the form of a pad 30 with gel beads containing glucose and potassium iodide, and a lower layer comprising a moist pad (or gel slab) 32 with calcium alginate beads containing entrapped glucose oxidase and calcium alginate beads containing entrapped lactoperoxidase.

Figure 4 is a variant of Figure 3 in which the upper layer comprises a semi-permeable sachet or gel slab 34 containing glucose and potassium iodide.

Further dressing components were prepared as follows.

### Preparation of an enzyme-containing pad

Loose woven cotton gauze is cut into a number of pieces approximately 10cm by 10cm and each piece is laid into a respective suitable flat bottomed container. 1% w/v alginic acid (also termed "alginate"; e.g. Manucol DM (Manucol DM is a Trade Mark), from CP Kelco) is prepared by dissolving the gel into an appropriate, heated aqueous solution. After cooling, enzyme is added to the alginate, to give a final concentration of 5 µg/ml glucose oxidase (GOX, from Boehringer Mannheim, cat. no.: 105147) or 10 µg/ml lactoperoxidase (LPO, from Sigma, cat. no.: L2005). 10mls of each enzyme-alginate solution is prepared, and poured evenly onto the individual cotton gauze pads. The gel is set by the addition of excess 10% w/v calcium chloride (CaCl₂) and allowed to stand for 10 minutes. The pads are then washed twice for 5 minutes each, in excess distilled/deionised water to remove the CaCl₂. The pads can then be stored in a humid environment to prevent drying out.

### Preparation of glucose-containing pad

Loose woven cotton gauze is cut to approximately 10cm by 10cm and is laid into a suitable flat bottomed container. 1% w/v agarose and 40% w/v glucose is dissolved into an appropriate aqueous solution and poured onto the gauze while still molten. The gel is allowed to set by cooling. The gel pad can then be stored in a humid environment to prevent drying out. To produce a pad containing glucose and potassium iodide, a similar procedure is followed, but after the glucose and agarose have been dissolved, potassium iodide (KI) is added to a final concentration of 10mM. The solution can then be poured, allowed to set and stored as above.

### Demonstration of the anti-microbial properties of an assembled composite dressing

### Example 1

Using standard practices, a 1.5% agarose microbial growth plate is prepared, but with the glucose replaced by fructose as the sole carbon source. The plates are typically in the region of 5mm thick. *Pseudomonas aeruginosa* was spread over the surface of the plate in an even "lawn". A pad containing LPO-alginate (prepared as described immediately above) of approximately 2cm² was placed onto the surface of the plate. Placed directly onto this pad is a 2cm² pad containing glucose-agar (prepared as described immediately above) (with or without KI). Finally, a 2cm² pad containing GOX-alginate (prepared as described immediately above) was layered onto the glucose-agar pad. A clearance zone around the pad stack can be clearly seen after 24 hours, showing the production and diffusion of active anti-microbial species, preventing the growth of the applied bacteria. Removal of any one of the 3 pads (control experiments) results in no clearance zone around the pad stack, showing that both enzymes and glucose all need to be present for the cascade to progress effectively.

### Example 2

Alternatively, to show the production of reactive oxidative species via the stacked enzyme system, a 1% w/v agar plate is case, that includes 1% soluble starch (e.g. ARCOS cat. no.: 177132500) and 10mM KI. The plate is allowed to set by cooling. The LPO-alginate pad, followed by the glucose-agar pad and finally the GOX-alginate pad can then be stacked as described above in Example 1. The production of the reactive oxidative species can be then visualised by the intense blue coloured chromogen produced by the well documented interaction of elemental iodine (the oxidative species oxidise the iodate to iodine) and starch. This coloration can be clearly seen after 5 minutes, with the intensity and spread building over time. After 30 minutes, the colour intensity has built to become a deep blue, indicating continued product formation. This shows that both reactive oxidative species and iodine are produced, both of which aid in the anti-microbial activity of the composite dressing.

### Example 3

A variation on Example 2 is to include a low level of GOX (0.25 µg/ml) in the LPO-alginate pad, to promote an initial production of reactive oxidative species. This utilises oxygen available in the gel, and is initiated by the glucose that is diffusing into the LPO-alginate pad. This reaction is limited, due to the availability of oxygen and will cease when the oxygen is depleted. To show the production of reactive oxidative species via the stacked enzyme system, a 1% w/v agar plate is cast, that includes 1% soluble starch (e.g. ARCOS cat. no.: 177132500) and 10mM KI. The plate is allowed to set by cooling. The combined LPO and GOX-alginate pad, followed by the glucose-agar pad and finally the GOX-alginate pad can the be stacked as described above. The accelerated production of the reactive oxidative species can be then visualised by the intense blue coloured chromogen produced by the well documented interaction of elemental iodine (the oxidative species oxidize the iodate to iodine) and starch. This coloration can be clearly seen after as little as 1 minute, with the intensity and spread building over time. The coloration will slow down noticeably after 15 minutes if the top GOX-alginate pad is not used. If the GOX-alginate pad is used as described above, the coloration will continue strongly, as seen in Example 2 above. After 30 minutes, the colour intensity has built to become a deep blue, indicating continued product formation. This shows that both reactive oxidative species and iodine are produced, both of which aid in the anti-microbial activity of the composite dressing.

## Claims

1. A skin dressing comprising oxidoreductase enzyme and peroxidase enzyme, wherein the enzymes are present in hydrated condition.

2. A dressing according to claim 1, wherein the oxidoreductase enzyme comprises glucose oxidase.

3. A dressing according to claim 1 or 2, wherein the peroxidase enzyme comprises lactoperoxidase.

4. A dressing according to any one of the preceding claims, wherein the enzymes are present in one or more hydrated gels.

5. A dressing according to claim 4, wherein the hydrated gel comprises an alginate gel.

6. A dressing according to claim 4 or 5, wherein enzymes are chemically conjugated to each other, chemically conjugated to other molecules or incorporated in a solid support.

7. A dressing according to claim 5 or 6, wherein the alginate gel is cast around a mechanical reinforcing structure.

8. A dressing according to any one of claims 1 to 4, wherein the hydrated gel comprises a shear-thinning gel.

9. A dressing according to any one of the preceding claims, wherein the dressing is of layered construction.

10. A dressing according to claim 9, comprising a first hydrated gel with oxidoreductase enzyme located in the vicinity of the outer parts of the dressing, i.e. remote from the skin in use.

11. A dressing according to claims 9 or 10, comprising a second hydrated gel with peroxidase enzyme located in the vicinity of the inner parts of the dressing, i.e. adjacent the skin in use.

12. A dressing according to any one of the preceding claims, including a layer of barrier material at the interface with the skin in use.

13. A dressing according to anyone of the preceding claims, including a source of substrate for the oxidoreductase enzyme.

14. A dressing according to claim 13, wherein the substrate is contained within an enclosure of a semi-permeable membrane material or within a gel slab.

15. A dressing according to claim 14, comprising a linear arrangement of linked slabs or panels, including a slab of the first hydrated gel, a slab of the second hydrated gel and a slab of substrate, with adjacent slabs linked by a respective foldable hinge portion.

16. A dressing according to claim 13, 14 or 15, wherein the source of substrate is located, in use, sandwiched between an upper layer of oxidoreductase enzyme and a lower layer of peroxidase enzyme.

17. A dressing according to any one of the preceding claims, further including a supply of iodide.

18. A dressing according to any one of the preceding claims, including a covering or outer layer for adhering the dressing to the skin of a human or animal subject.

19. A dressing according to claim 18, wherein the covering includes a window in or through which can be seen indicator means that indicates when the dressing chemistry is active.

20. A dressing according to claim 18 or 19, wherein immobilised catalase enzyme is provided on the inner surface of the covering.

21. A dressing according to any one of the preceding claims, in the form of a multi-part system, with different elements separately packaged.

22. A dressing according to claim 21, wherein the oxidoreductase enzyme and a source of substrate for the oxidoreductase enzyme are in separate packages.
